# EUROPEAN PATENT SPECIFICATION

(11) **EP 3 004 847 B1**
(45) Date of publication and mention of the grant of the patent: **10.04.2019**
(21) Application number: 14727459.1
(22) Date of filing: 28.05.2014
(51) Int. Cl.: G01N 21/64, G02B 21/16, G02B 27/56

(54) **FLUORESCENCE IMAGING SYSTEM FOR TISSUE DETECTION**
FLUORESZENZABBILDUNGSSYSTEM ZUR GEWEBEERKENNUNG
SYSTÈME D'IMAGERIE PAR FLUORESCENCE POUR DÉTECTION DE TISSU

(30) Priority: 03.06.2013 US 201361830544 P
(43) Date of publication of application: 13.04.2016
(73) Proprietor: Ventana Medical Systems, Inc., Tucson, Arizona 85755 (US)
(72) Inventor: BAMFORD, Pascal, Oro Valley, Arizona 85755 (US); DIETZ, Lou, Mountain View, California 94041 (US); LITTLE, Elizabeth, San Jose, California 95125 (US)
(74) Representative: Richardt Patentanwälte PartG mbB
(86) International application number: PCT/EP2014/061052
(87) International publication number: WO 2014/195204

(56) References cited:
- EP-A2- 2 290 352
- WO-A1-2013/035028
- US-A- 5 633 724
- US-A- 5 982 534
- US-A1- 2008 212 866
- US-A1- 2010 321 696
- VO-DINH T ET AL: "DEVELOPMENT OF A MULTIARRAY BIOSENSOR FOR DNA DIAGNOSTICS", INSTRUMENTATION SCIENCE & TECHNOLOGY, TAYLOR & FRANCIS INC, US, vol. 26, no. 5, 1 November 1998 (1998-11-01), pages 503-514, XP000788905, ISSN: 1073-9149, DOI: 10.1080/10739149808000917
- ANNE SENTENAC ET AL: "High-resolution total-internal-reflection fluorescence microscopy using periodically nanostructured glass slides", JOURNAL OF THE OPTICAL SOCIETY OF AMERICA A, vol. 26, no. 12, 1 December 2009 (2009-12-01), page 2550, XP055132818, ISSN: 1084-7529, DOI: 10.1364/JOSAA.26.002550

## Description

### TECHNICAL FIELD

This disclosure relates to systems for imaging specimens. In particular, the disclosure relates to fluorescence imaging systems for tissue detection.

### BACKGROUND

Conventional fluorescence microscope scanners, used for whole slide imaging or for rare cell detection, typically scan a whole microscope slide at a low magnification (e.g., less than 4X magnification) by using a low magnification microscope objective positioned on the front side of the slide and a light source positioned on the backside of the slide. Light from the light source can travel through the slide to illuminate specimens carried on a front surface of the slide. To produce an image of the whole slide, small areas of the slide are sequentially imaged to produce a set of sub-images. The sub-images can be combined to produce a composite image of the whole slide for interpretation by, for example, a pathologist. Unfortunately, the overall imaging time can take several minutes to several hours depending on the number of specimens on the slides, locations of specimen(s) on the slide, level of magnification, and analysis and interpretation to be performed. Additionally, acquiring the sub-images often requires complicated and expensive microscopy equipment capable of accurately moving a camera and objective relative to the slide. Excitation light from the light source passes once through stained tissue specimens (e.g., using epi-illumination or wide area illumination) to produce fluorescence emissions. The emission light is captured by a camera. Unfortunately, the specimens absorb a small fraction of the excitation light resulting in relatively weak fluorescence emissions in comparison to the excitation light, resulting in a low signal-to-noise ratio. This makes it difficult to interpret or analyze the fluorescence image.

US 5,982,534 discloses a specimen illumination apparatus wherein light is transmitted by total internal reflection for illuminating a specimen located between a coverslip and a slide.

EP 2 290 352 A2 discloses a darkfield illumination system wherein excitation light is coupled to the lateral surface of a slide glass.

### OVERVIEW OF TECHNOLOGY

The present invention provides an imaging system as defined in claim 1, and a method for imaging a specimen as defined in claim 5. At least some embodiments of the technology include an imaging system that includes one or more light sources, cameras, and imaging components (e.g., lenses) that cooperate to evenly illuminate specimens carried on a microscope slide and to capture a single image containing all the specimens. The light sources output excitation light for causing fluorescing of the specimens (e.g., fluorescing of fluorophores, tissue, etc.). The slide serves as a light guide to efficiently deliver the excitation light to the specimens. The excitation light excites the specimens at wavelength(s) or waveband(s) in any desired portion (e.g., the visible portion) of the spectrum detectable by the camera(s). The excitation light is at wavelength(s) or waveband(s) that are different from the wavelength(s) or waveband(s) of the fluorescence emission. In some embodiments, the tissue fluoresces in the visible portion of the spectrum to provide whole slide imaging with relatively low cost cameras and lenses.

In some embodiments, a single whole slide image can be used to locate specimens, count specimens, and acquire other information about the specimen(s) and/or slide. Based on the acquired information, subsequent imaging can be performed. For example, high resolution imaging (e.g., scanning) can be limited to the areas of the slide carrying specimens. Thus, high resolution images of all areas of interest can be captured in a relatively short period of time. This can increase the throughput of the imaging system, reduce diagnostic times, etc. In some embodiments, a single image of the whole slide can be acquired very rapidly. For example, a whole slide image can be acquired in less than 3 seconds, 2 seconds, 1 second, or less.

The excitation light is constrained by the microscope slide and/or coverslip. Total internal reflection is achieved to limit or prevent an appreciable amount of excitation light from reaching the camera. The excitation light travels multiple times back and forth between the slide and the coverslip, resulting in high intensity illumination. The total internal reflection also minimizes or limits changes (e.g., decreases) in intensity associated with the distance between the light source and tissue because the excitation light undergoes multiple reflections to generally homogenize the illumination and provide very uniform intensity across the entire slide. The total internal reflection also limits or minimizes stray illumination excitation light reflected towards the camera. This prevents overwhelming relatively weak fluorescence emissions from tissue sample(s) with the excitation light and eliminates the need for complicated or expensive filters (e.g., filters for blocking excitation light).

The excitation light causes fluorescence, including, without limitation, auto-fluorescence of the tissue sample, fluorescence of fluorophores (including fluorochromes, fluorescent reagents, and/or fluorescent dyes), and/or other mechanisms of producing fluorescence. With fluorescently stained tissue samples, UV light can excite fluorophores in the tissue. With chromogenic stained tissue samples, the UV light can cause chromogen fluorescence and/or auto-fluorescence of the tissue itself. For example, tissue lightly stained with chromogen(s) can be located based on chromogen fluorescence, tissue auto-fluorescence, or both. In some procedures, auto-fluorescence of the tissue alone is used to locate the tissue. Other types of light, including non-UV light sources, can also be used. The light sources are arrays of light emitting diodes (LEDs), such as relatively low cost ultraviolet LEDs or other types of LEDs.

The imaging system includes a light generator positionable next to at least one edge of a microscope slide. The light generator can deliver excitation light to the edge of the slide such that the light is internally reflected by the slide and/or coverslip to illuminate one or more tissue samples carried on the microscope slide. The internally reflected light can cause a fluorescence emission from the slide. In some embodiments, the fluorescence emission from the slide can be produced by auto-fluorescing of the tissue itself. In other embodiments, the fluorescence emission from the slide can be produced by fluorescing of one or more fluorophores associated with (e.g., bound to) the tissue.

An image capturing device captures the emission to produce a slide image. Most of the radiation captured by the image capturing equipment is light of the fluorescence emission. The slide and coverslip cooperate to internally reflect the excitation light such that most of the light reaching the image capturing device is the fluorescence emission. The excitation light (i.e., light delivered into the slide and/or coverslip) that reaches the image capturing device can be kept at or below a threshold level. In some procedures, an image of the slide is produced based on both the fluorescence emission and excitation light. The tissue sample can scatter the excitation light such that some excitation light reaches the image capture device. The excitation light summed with the fluorescence emission can be used locate the tissue.

In some embodiments, an imaging system is configured to capture a single wide-area image used for obtaining information about a slide. The information can include, without limitation, the presence of tissue sample(s), the number of tissue sample(s), spatial information (e.g., positions of the tissue samples, spacing between tissues samples, etc.), shape/size of the tissue samples, tissue type, or other desired information. In one embodiment, additional imaging can be performed based on the information. The additional imaging can be higher resolution imaging of areas of interest of the slide to, for example, image only areas with tissue.

The imaging system is configured to detect tissue situated on a microscope slide that has an upper surface, a lower surface, and a plurality of edges. The tissue can be located on the upper surface of the microscope slide. The imaging system includes a light source, a camera, and an imaging lens. The light source is oriented proximate to opposite edges of the microscope slide so as to direct light to the edges of the slide. The light undergoes total internal reflection between a lower surface of the microscope slide and a coverslip situated on the slide. The total internal reflection of the light causes a fluorescence emission from the specimen.

The camera, in some embodiments, can capture a single whole slide image. For example, the camera and imaging lens can be configured such that the camera captures a single fluorescent-enhanced whole slide image. The imaging lens is positioned to direct radiation (e.g., a fluorescence emission) onto the camera. The camera can be a thumbnail camera or other device capable of producing a desired image.

The light source, in some embodiments, comprises an ultraviolet (UV) light source. The UV light source can comprise UV LEDs that are proximate to opposite edges of the slide. The light source includes a pair of arrays of light emitting diodes. The light source can also be part of a light generator with a light baffle positioned to block excitation light to prevent direct illumination of the camera. The light baffle can be an opaque plate positioned between the light source and the camera and/or imaging lens.

A fluorophore can be bound to or accumulated in the tissue. In some embodiments, the fluorophore comprises an organic fluorophore, quantum dot(s), or other substance(s) capable of producing fluorescence emission(s). In some embodiments, two or more fluorophores are bound to the tissue. The light source can include one or more light sources capable of emitting light at wavelengths for stimulating each of the fluorophores. For example, if the sample includes two fluorophores, the light source can include two light emitters, each capable of exciting one of the fluorophores.

At least some embodiments are a method for imaging a microscope slide, as defined in claim 5. The method includes directing light into an edge of the microscope slide at an angle sufficient to trigger total internal reflection of the light between the microscope slide and the coverslip covering the slide. The light emitted from the slide and/or the coverslip is directed towards a camera. The emitted light can be from fluorescing tissue, fluorescing fluorophores, or combinations thereof. The camera can capture the emitted light and generate one or more images of the slide. The light is internally reflected by the coverslip (e.g., an upper surface or a lower surface of the coverslip) and a lower surface of the microscope slide. As such, the light can repeatedly pass through the tissue to substantially uniformly illuminate the tissue. A fluorescence emission from the tissue can be directed from the slide and/or coverslip towards the camera.

A microscope can include the imaging systems disclosed herein. The microscope can include additional features including, without limitation, one or more filters, imaging optics, controllers, readers, or the like. In some embodiments, the imaging systems or components thereof can be incorporated into standard microscopes.

### BRIEF DESCRIPTION OF THE DRAWINGS

Non-limiting and non-exhaustive embodiments are described with reference to the following drawings. The same reference numerals refer to like parts or acts throughout the various views, unless otherwise specified. In the following, only embodiments being in full agreement with the definitions of the claims form embodiments according to the invention.
**Figure 1** is a front view of an imaging system in accordance with one embodiment.
**Figure 2** is a top plan view of a light generator and a specimen-bearing microscope slide.
**Figure 3** is an image of a whole specimen-bearing microscope slide.
**Figure 4** is a side view of a portion of an imaging system and a microscope slide in accordance with one embodiment.
**Figure 5** is a detailed view of a light source and end portions of a microscope slide and a coverslip.
**Figure 6** is a flow chart of a method of imaging a microscope slide in accordance with one embodiment.
**Figure 7** is a front view of an automated imaging system in accordance with one embodiment.
**Figure 8** is a top plan view of a light generator positioned to illuminate a specimen carried by a microscope slide in accordance with one embodiment.
**Figure 9** is a top plan view of a light generator positioned to deliver light to opposing edges of a microscope slide in accordance with one embodiment.

### DETAILED DESCRIPTION OF TECHNOLOGY

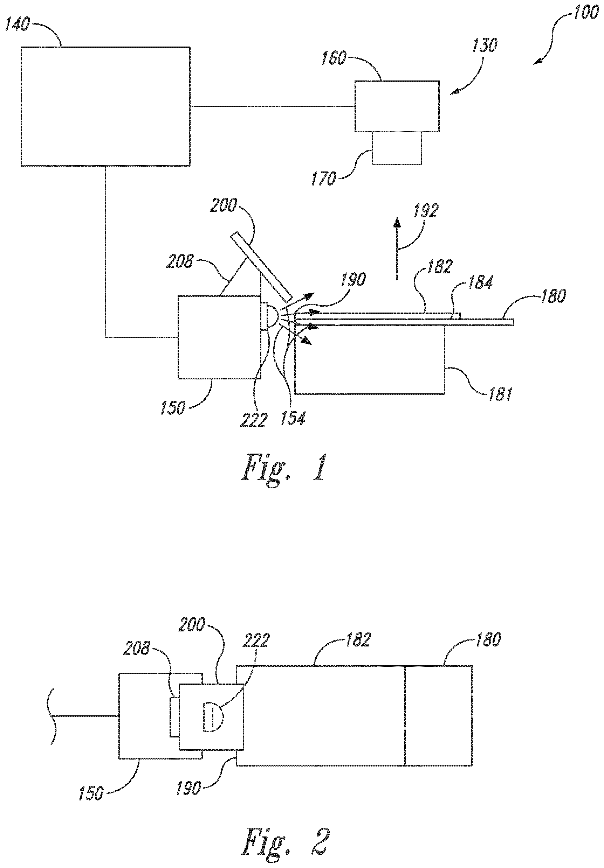

Imaging systems and associated methods for imaging microscope slides are described herein. The imaging systems can include a light source that directs excitation light towards a microscope slide such that the light undergoes internal reflection between a surface of the slide and a coverslip, which covers a specimen situated on the slide. The microscope slide and/or coverslip can serve as waveguides to efficiently illuminate the specimen to cause fluorescing of the specimen. The excitation light can undergo total internal reflection to provide a substantially spatially uniform distribution of light to excite, without limitation, the tissue, fluorophores, or other substance(s) to enable rapid slide imaging for automated tissue detection/analysis. A person skilled in the relevant art will understand that the technology may have additional embodiments and that the technology may be practiced without several of the details of the embodiments described below with reference to Figures 1-9.

Figure 1 is a front view of an imaging system 100 in accordance with one embodiment. The imaging system 100 can include an imager 130, a computing device 140, and a light generator 150. The imager 130 can include an image capturing device 160 and imaging optics in the form of an imaging lens 170. The light generator 150 can output excitation light (represented by arrows 154) that causes fluorescence of a specimen carried by a microscope slide 180 ("slide 180") located on a slide holder 181. The fluorescence can be, for example, fluorescence of a fluorophore bound to tissue, fluorescence of tissue itself, etc. The slide 180 and a coverslip 182 can serve as waveguides that cooperate to illuminate the entire specimen (or multiple specimens) to produce an emission (represented by arrow 192). The imaging optics 170 can direct the emission 192 towards the image capturing device 160, which in turn produces an image of the slide 180. The computing device 140 can analyze the image to determine, for example, the number of tissue samples carried by the slide 180, position of the tissue sample(s), shapes of the tissue sample(s), and other information about the tissue samples.

Figure 2 is a top plan view of the light generator 150, slide 180, and coverslip 182. Referring to Figures 1 and 2 together, the light generator 150 can be positioned proximate to an edge 190 (e.g., a side surface, a corner, combinations thereof, etc.) of the slide 180 and/or coverslip 182. The light 154 can strike the edge 190, travel through the slide 180 and coverslip 182, and be internally reflected by the slide 180 and/or coverslip 182. In some embodiments, the light can undergo total internal reflection to deliver light to substantially an entire interface (e.g., between the slide 180 and coverslip 182) or coverslipped area 184 (Figure 1) of the slide 180. In some embodiments, the total internal reflection of the light causes the excitation energy to be delivered to at least about 90% of the coverslipped area 184. In one embodiment, the excitation energy is delivered to at least about 95% of the coverslipped area 184 to locate any tissue on the the coverslipped area 184.

A light baffle 200 can prevent light 154 from being captured by the image capturing device 160. In some embodiments, the light baffle 200 prevents light 154 from directly impinging on the imager 130 to minimize or limit noise associated with the excitation light. As shown in Figure 1, the light baffle 200 can be positioned directly between a light source 222 and imaging optics 170. The position, size, and optical characteristics of the light baffle 200 can be selected to block substantially all of the excitation light 154 outputted towards the image capturing device 160.

A coupler 208 can couple the light baffle 200 to the light generator 150. Alternatively, the light baffle 200 can be mounted to the imager 130 to allow convenient replacement of the light generator 150. Additionally or alternatively, one or more filters can be used to filter light outputted by the light generator 150 while allowing the emission 192 (Figure 1) to reach the image capturing device 160. The filters can be coupled to or incorporated into the imaging optics 170 and/or image capturing device 160. If the light generator 150 outputs a beam of light (e.g., a laser beam), the light baffle 200 and filters can be eliminated.

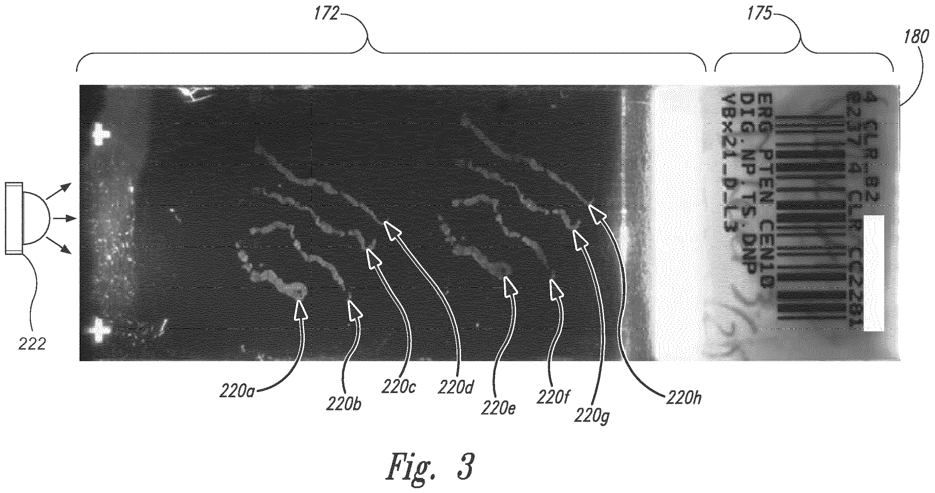

Figure 3 is an image of the whole slide 180 carrying an array of specimens. The specimens are eight tissue samples 220a-h (collectively "samples 220") situated on the slide 180. The samples 220 in Figure 3 are spaced apart prostate samples, but other types of tissue samples can be imaged. The tissue samples 220 can be fluorescently stained with, for example, fluorophores. The image of Figure 3 shows a mounting region 172 and a label region 175 of the slide 180. The mounting region 172 may include most of the slide 180 or approximately a 25 mm x 50 mm area of the slide 180. Referring to Figures 1 and 3, the computing device 140 (Figure 1) can detect all of the samples 220 (Figure 3) based on the single fluorescence-enhanced image. Additional imaging of one or more of the samples 220 can be performed.

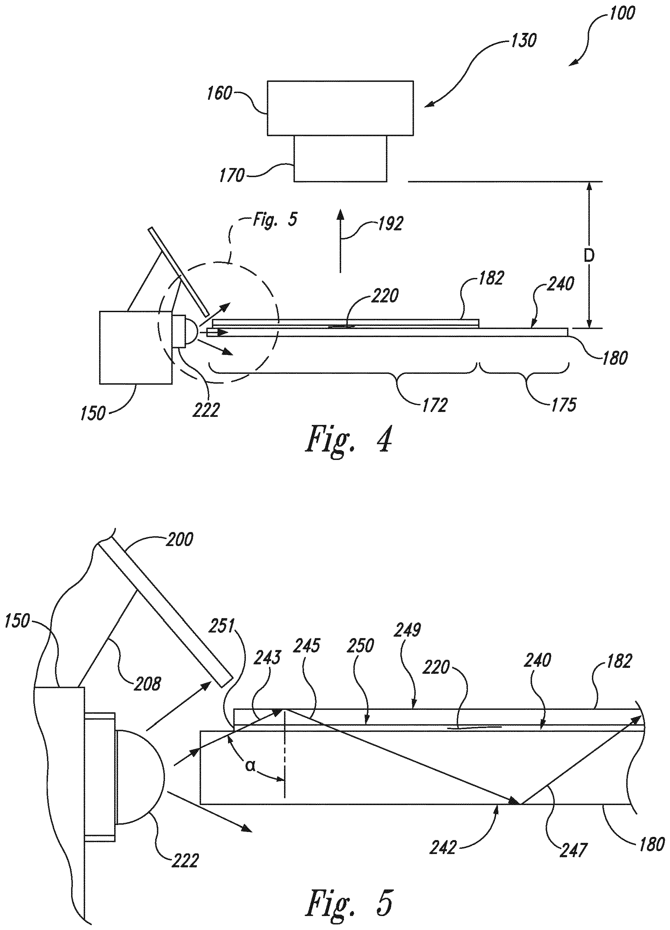

Figure 4 is a front view of components of the imaging system 100 in accordance with one embodiment. The coverslip 182 is proximate to the sample 220, which is situated on an upper surface 240 on the slide 180. The image capturing device 160 can capture a single fluorescence-enhanced image of the whole slide 180. In other procedures, the image capturing device 160 and imaging lens 170 can cooperate to capture an image of the mounting region 172 of the slide 180. A separate image of the label region 175 can be captured using white light. The two images can be overlayed or otherwise combined to produce a composite whole slide image. Thus, the imaging system 100 can produce a single whole slide image or a composite whole slide image.

The image capturing device 160 can include a camera, such as an IDS UI-1495LE thumbnail camera from Phase 1 Technology Corporation (Deer Park, NY) or other thumbnail camera. The cameras can include, without limitation, one or more sensors, such as a charge-coupled device (CCD) and/or complementary metal-oxide-semiconductor (CMOS) device. The configuration and resolution of the sensors can be selected based on the desired characteristics of the images. In some embodiments, the imaging optics 170 are incorporated into the image capturing device 160. Additionally, the image capturing device 160 can capture the image in a relatively short period of time. In some embodiments, a single image containing all of the tissue samples on the slide 180 can be captured in less than about 3 seconds, 2 seconds, or 1 second. If the computing device 140 (Figure 1) includes a Universal Serial Bus (USB) port, the image capturing device 160 can be a USB camera connectable to the computing device 140 via a USB cable. Other wired or wireless connections can provide communication between the imaging capture device 160 and the computing device 140.

The imaging optics 170 can include, without limitation, one or more microscope objectives, lenses (e.g., focusing lenses), sensor focus lens groups, or other optical components for achieving a desired magnification, if any. Referring to Figure 1, the computing device 140 can command the imaging optics 170 to increase or decrease magnification, adjust focus, or otherwise select the characteristics of captured images.

Referring to Figures 1 and 4, the relative position between the slide 180 and imager 130 can be adjusted to ensure that the image capturing device 160 receives the emission 192. In some routines, a distance D of Figure 4 is in a range of about 170 mm to about 190 mm. For example, the distance D can be about 180 mm to capture a single image of substantially the entire microscope slide 180. Other distances D can also be used.

Figure 5 is a detailed view of a portion of the light generator 150, slide 180, and coverslip 182. The light source 222 can include, without limitation, one or more LEDs (e.g., surface emitting LEDs, edge emitting LEDs, super luminescent LEDs, or the like), laser diodes, electroluminescent light sources, incandescent light sources, cold cathode fluorescent light sources, organic polymer light sources, lamps, inorganic light sources, or other suitable light emitters. The illustrated light source 222 can output wavelength(s) and/or waveband(s) that correspond with, or at least overlap with, the wavelength(s) or waveband(s) that excite, alter, or otherwise activate a reagent (e.g., stain, fluorophores, etc.) and/or tissue to cause fluorescing. For example, excitation light can be the light of a particular wavelength(s) and/or waveband(s) necessary and/or sufficient to excite an electron transition to a higher energy level. In one example, excitation light has a particular wavelength necessary and/or sufficient to excite a fluorophore bound to the tissue to a state such that the fluorophore will emit a different (such as a longer) wavelength of light than the wavelength of the excitation light, to produce fluorescence. Fluorescence can be the emission of radiation by an atom or molecule passing from a higher to a lower state. Fluorescence can occur when the atom or molecule absorbs the excitation energy and then emits the energy as radiation, such as visible radiation. In some embodiments, the light source 222 can output an ultraviolet stimulus beam (e.g., a beam in a wavelength range of about 370 nm +/- 20 nm) to excite a fluorophore that can be, without limitation, semiconductor nanocrystal quantum dot, fluorescent stain, or other fluorophore bound to the tissue 220, or other naturally-occurring molecules in the tissue that cause auto-fluorescence.

The slide 180 can be a substantially flat substrate capable of carrying samples for examination. For example, the slide 180 can be a generally rectangular piece of transparent material having the flat upper surface 240 and a lower surface 242. The optical characteristics of the slide 180 and/or coverslip 182 can be selected to achieve desired internal reflectance. Light rays (represented by arrows 243, 245, 247) internally reflected by the lower surface 242 and either an upper surface 249 or a lower surface 250 of the coverslip 182. The internally reflected light can travel through the tissue 220 multiple times to provide high intensity illumination. For example, the light can experience total internal reflection to limit or minimize intensity decreases associated with light traveling through the tissue 220. The internally reflected light can repeatedly travel through the tissue 220 to provide generally uniform illumination across the entire tissue 220 to limit or prevent variations in intensity based on the distance between the light source and the tissue. Advantageously, the excitation light 243, 245, 247 can be constrained within the microscope slide 180 and/or coverslip 182 to minimize or limit excitation illumination directed towards the image capturing device 160, which tends to overwhelm the relatively weak fluorescence emission from the tissue 220. The indexes of refraction of the microscope slide 180 and coverslip 182 can be selected such that the angles the excitation light strikes the upper surface 249 of the coverslip 182 and the slide lower surface 242 are larger than the critical angle (i.e., the critical angle with respect to an axis normal to the corresponding surfaces 242, 249). In some embodiments, the index of refraction of the microscope slide 180 can be about 1.54 at a wavelength of 365 nm. Air with a refractive index of 1 can surround the slide 180, and the critical angle α can be about 40.6 degrees. In some embodiments, the indexes of refraction of the slide 180, coverslip 182, and/or tissue/mounting medium 251 can be generally equal to one another. For example, a ratio of the refractive index of the slide 180 to the refractive index of the coverslip 182 can be in a range of about 0.9 to about 1.1. Other ratios and indexes of refraction can also be used.

In one embodiment, the slide 180 can be a standard microscope slide made of glass, such as borosilicate glass (e.g., BK7 glass). The slide 180 can have a length of about 3 inches (75 mm), a width of about 1 inch (25 mm), and a thickness of about 1 mm. Slides made of different materials and with different dimensions can be used. The coverslip 182 can also be made of glass (e.g., borosilicate glass) or other optically transparent or semi-transparent materials (e.g., plastics or polymers). Both the slide 180 and coverslip 182 can be substantially flat substrates. The term "substantially flat substrate" refers, without limitation, to any object having at least one substantially flat surface, but more typically to any object having two substantially flat surfaces on opposite sides of the object, and even more typically to any object having opposed substantially flat surfaces, which opposed surfaces are generally equal in size but larger than any other surfaces on the object.

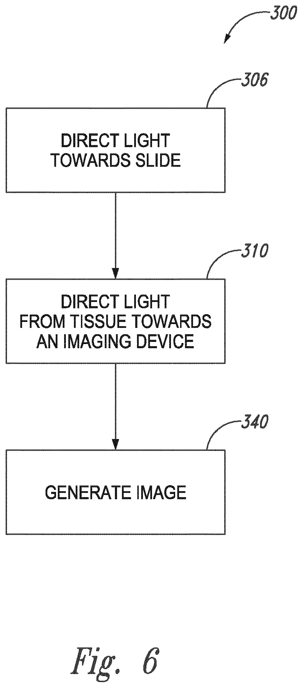

Figure 6 is a flow chart of a method 300 for imaging a slide. Generally, light is directed into the slide such that the light is internally reflected by the slide and/or coverslip. The reflected light illuminates specimen(s) carried by the slide to produce one or more fluorescence emissions used to generate an image of the slide. The image can be used with automated tissue detection or analysis routines. The method 300 is discussed in connection with Figures 1-5, but it can be performed with other imaging systems.

At stage 306, a microscope slide can be loaded onto the holder 181 of Figure 1. The slide 180 can carry one or more specimens treated with a reagent comprising one or more fluorphores. The fluorphores can include, without limitation, one or more organic fluorophores, quantum dots, DNA binding moieties, or other substances capable of, for example, fluorescently defining and delineating characteristics or features (e.g., tissue sample boundaries, cellular structures, etc.) of specimens. Quantum dots can provide a photostable fluorescent signal or other type of fluorescence emission. A broad-range absorption spectra (e.g., quantum dot absorption spectra can span the upper and lower ultraviolet regions and can extend into the visible region, depending upon the size of the quantum dots) and high quantum yields (e.g., > 30%, > 50%, or > 80%) can be used for fluorescent staining of nuclei in tissue in conjunction with, for example, assays (e.g., fluorescent HER2 and TMPRSS2-ERG assays). In some protocols, formalin-fixed, paraffin embedded histological tissue sections can be prepared according to fluorescence in-situ hybridization (FISH) protocols (e.g., protocols from Ventana Medical Systems, Inc. (Tucson, AZ) FISH protocols) involving, for example, treatment with semiconductor nanocrystal quantum dot (QDot) detection and counterstained with fluorescent stain 4',6-diamidino-2-phenylindole (DAPI). Figure 3 shows prostate samples 220 stained with DAPI and QDot reagents. QDot detection and DAPI fluorescence can be produced with an ultraviolet light (UV light) in a wavelength range of about 370 nm +/- 20 nm. In some routines, UV light from the light source 222 can cause simultaneous multiplex excitation of UV-absorbing nuclear counterstains (such as DAPI) as well as multiplexed QDot probes.

QDots can be nanoscale particles that exhibit size-dependent electronic and optical properties due to quantum confinement and can be constructed of, for example, one or more semiconductor materials (e.g., cadmium selenide and lead sulfide), crystallites (e.g., crystallites grown via molecular beam epitaxy), etc. A variety of QDots having various surface chemistries and fluorescence characteristics are commercially available from Invitrogen Corporation, Eugene, OR (see, for example, U.S. Patent Nos. 6,815,064, 6,682,596 and 6,649,138). Quantum dots are also commercially available from Evident Technologies (Troy, NY). Other quantum dots include alloy quantum dots, such as ZnSSe, ZnSeTe, ZnSTe, CdSSe, CdSeTe, ScSTe, HgSSe, HgSeTe, HgSTe, ZnCdS, ZnCdSe, ZnCdTe, ZnHgS, ZnHgSe, ZnHgTe, CdHgS, CdHgSe, CdHgTe, ZnCdSSe, ZnHgSSe, ZnCdSeTe, ZnHgSeTe, CdHgSSe, CdHgSeTe, InGaAs, GaAlAs, and InGaN quantum dots.

At stage 310 of Figure 6, light is directed to towards the microscope slide. Referring to Figures 1-5, the excitation light can travel through the microscope slide 180 and/or coverslip 182 as discussed in connection with Figures 4 and 5. In fluorescence imaging, the light can stimulate the fluorescence reagent to produce a fluorescence emission for high sensitivity to, for example, enhance image processing for detecting specimens, identifying characteristic features of the specimens, or other image processing.

At stage 340 of Figure 6, the imager 130 can generate an image that is transmitted to the computing device 140 (Figure 1). The computing device 140 can analyze the image and command the imager 130 based on the image analysis. If the imager 130 is a microscope capable of providing different amounts of magnification, the imager 130 can capture images at different magnifications.

The method 300 of Figure 6 can be performed to detect a wide range of different samples. The term "sample" refers to any liquid, semi-solid or solid substance (or material) in or on which a target can be present. In particular, a sample can be a biological sample or a sample obtained from a biological material. Examples of biological samples include tissue samples and cytology samples. In some examples, the biological sample is obtained from an animal subject, such as a human subject. A biological sample is any solid or fluid sample obtained from, excreted by or secreted by any living organism, including without limitation, single celled organisms, such as bacteria, yeast, protozoans, and amebas among others, multicellular organisms (such as plants or animals, including samples from a healthy or apparently healthy human subject or a human patient affected by a condition or disease to be diagnosed or investigated, such as cancer). For example, a biological sample can be a biological fluid obtained from, for example, blood, plasma, serum, urine, bile, ascites, saliva, cerebrospinal fluid, aqueous or vitreous humor, or any bodily secretion, a transudate, an exudate (for example, fluid obtained from an abscess or any other site of infection or inflammation), or fluid obtained from a joint (for example, a normal joint or a joint affected by disease). A biological sample can also be a tissue sample obtained from any organ or tissue (including a biopsy or autopsy specimen, such as a tumor biopsy) or can include a cell (whether a primary cell or cultured cell) or medium conditioned by any cell, tissue or organ. In some examples, a biological sample is a nuclear extract. In some examples, a biological sample is bacterial cytoplasm. In other examples, a sample is a test sample. For example, a test sample is a cell, a tissue or cell pellet section prepared from a biological sample obtained from a subject. In an example, the subject is one that is at risk or has acquired a particular condition or disease.

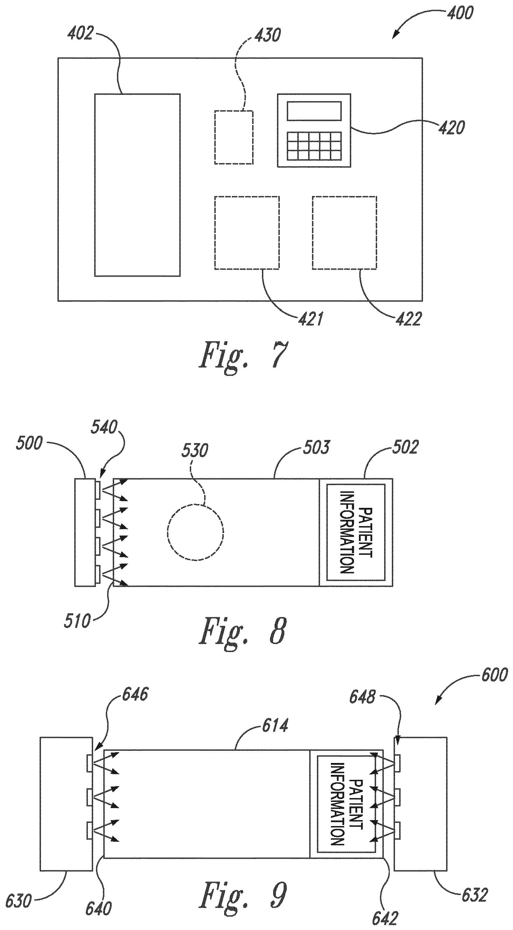

Figure 7 is a front view of an automated imaging system 400 in accordance with one embodiment. An access door 402 can be opened to load coverslipped slides into the imaging system 400. After loading the slides, the access door 402 can be closed to begin processing. A transport device 430 (shown schematically in phantom line) can transport the slides between imaging systems 421, 422 (shown schematically in phantom line). The imaging system 421 can produce images for tissue detection, and the imaging system 422 can produce images for tissue analysis (e.g., high resolution images for interpretation).

In some embodiments, a controller 420 can command the imaging system 421 to capture low resolution whole slide images. The imaging system 421 can be similar or identical to the imaging system 100 of Figure 1. The controller 420 can analyze the low resolution images to detect tissue samples and can command the imaging system 422 to capture higher resolution images of the detected tissue samples. Advantageously, the imaging system 400 can obtain high resolution images of all of the tissue samples (or identified tissue samples) without scanning the entire slide (i.e., areas of the slide without tissue samples), thereby limiting overall imaging times and increasing throughput. The overall imaging times can vary between sides because the number and sizes of specimens on different slides may vary.

In some procedures, fluorescence-enhanced images from the imaging system 421 can be used to obtain information about the specimen(s). The imaging system 422 can then capture non-fluorescence-enhanced images, such as brightfield images of the slide. The tissue samples can thus be stained with reagents suitable for fluorescence and/or brightfield imaging. The term "reagent" refers to biological or chemical substances which, when applied to targeted molecules or structures in tissue, renders the tissue detectable using an instrument. Stains include, without limitation, detectable nucleic acid probes, antibodies, hematoxylin, eosin, and dyes (*e.g.,* iodine, methylene blue, Wright's stain, etc.). In some procedures, the specimen can be stained with a fluorophore reagent for imaging with the imaging system 421 and a non-fluorophore reagent for imaging with the imaging system 422. In some cases, the specimen has innate auto-fluorescent molecules that can be imaged by the imaging system 421 without the addition of any reagents. In some cases, the brightfield reagents also beneficially cause fluorescence, which can be imaged by imaging system 421, without the addition of other fluorescent reagents. In some embodiments, the imaging system 400 can be a scanner, such as the iScan Coreo scanner from Ventana Medical Systems, Inc. (Tucson, AZ). The imaging system 421 can be installed in the scanner to image slides prior to scanning the tissue areas at a desired magnification (e.g., 4X, 10X, 20X, or 40X magnification). The imaging systems or components disclosed herein can also be incorporated into other types of imaging equipment, including standard scanners.

Referring to Figure 7, the controller 420 can be communicatively coupled to and command the imaging systems 421, 422 and transport device 430. The controller 420 can generally include, without limitation, one or more computers, central processing units, processing devices, microprocessors, digital signal processors (DSPs), application-specific integrated circuits (ASICs), readers, and the like. To store information (e.g., executable instructions), the controller 420 can include, without limitation, one or more storage elements, such as computer readable media, volatile memory, non-volatile memory, read-only memory (ROM), random access memory (RAM), or the like. The controller 420 can include one or more processors that are programmed with a series of computer-executable instructions that are stored on a non-transitory, computer readable media. The stored computer-executable instructions can include detection programs, optimization programs, calibration programs, image processing programs, or other executable programs. Detection programs can be executed to identify boundaries or edges of specimens and/or detect dots. Optimization programs can be executed to optimize performance (e.g., decrease imaging times, enhance imaging consistency, or the like). The processing may be optimized by determining, for example, an optimum boundary detection routing to (1) increase imaging speeds and throughput (e.g., increase the number of slides processed in a certain length of time) and (2) accurately detect samples.

The transport device 430 of Figure 7 can include, without limitation, one or more slide handlers, slide trays, slide holders, or the like. Slide handlers can include, but are not limited to, slide manipulators, X-Y-Z transport systems, robotic systems, or other automated systems capable of receiving and transporting slides. A robotic system can include, without limitation, one or more pick and place robots, robotic arms, or the like.

Figure 8 is a top plan view of a light generator 500 positioned to deliver light to a microscope slide 502 and/or coverslip 503 in accordance with one embodiment. The light generator 500 can include an array of spaced apart light sources 504 proximate to an edge 510 of the microscope slide 502 and/or coverslip 503. A tissue sample 530 (illustrated in phantom line) can include multiple fluorophores, each excitable by light outputted by at least one of the light sources 540. In some embodiments, two or more fluorophores are bound to the tissue sample 530. The light generator 500 can emit light at two or more wavelength(s) or waveband(s) to stimulate each of the fluorophores. As such, the number, positions, and wavelength(s)/waveband(s) of light from the light sources 540 can be selected based on the characteristics of the tissue and/or fluorophores.

Figure 9 is a top plan view of a light generator 600 positioned to deliver light to opposing edges 640, 642 of a microscope slide 614 in accordance with one embodiment. The light generator 600 can include a pair of light generators 630, 632 oriented proximate to the edges 640, 642, respectively. Each light generator 630, 632 includes an array of light sources 646, 648. In the illustrated embodiment, each array includes three light sources but any desired number of light sources can be used. Advantageously, the effects associated with transmission losses can be minimized or limited by using light in different directions through the slide 614. Any number of light sources can surround a slide 614 (and coverslip) to obtain the desired uniform illumination.

From the foregoing, it will be appreciated that specific embodiments of the invention have been described herein for purposes of illustration, but well-known structures and functions have not been shown or described in detail to avoid unnecessarily obscuring the description of at least some embodiments of the invention. For example, the light blocking baffle can be replaced with one or more filters that block specific wavelength(s) or waveband(s). In some embodiment, the imaging optics disclosed herein can block wavelengths of light from the light generator while allowing the passage of the wavelength of the emission. The imaging systems disclosed herein can be part of or incorporated into a wide range of different types of standard microscope. In some embodiments, the imaging system 100 is a microscope. Where the context permits, singular or plural terms may also include the plural or singular term, respectively. Unless the word "or" is associated with an express clause indicating that the word should be limited to mean only a single item exclusive from the other items in reference to a list of two or more items, then the use of "or" in such a list shall be interpreted as including (a) any single item in the list, (b) all of the items in the list, or (c) any combination of the items in the list. The singular forms "a," "an," and "the" include plural referents unless the context clearly indicates otherwise. Thus, for example, reference to "a specimen" refers to one or more specimens, such as two or more specimens, three or more specimens, or four or more specimens.

In general, in the following claims, the terms used should not be construed to limit the claims to the specific embodiments disclosed in the specification and the claims, but should be construed to include all possible embodiments to which such claims are entitled. Accordingly, the invention is limited by the scope of the appended claims.

## Claims

1. An imaging system (100) for tissue detection of a specimen (220) situated on a microscope slide (180, 502) having an upper surface (240), a lower surface (242), and a plurality of edges, the specimen (220) is located on the upper surface (240), the imaging system (100) comprising:
a light source (222) comprising a pair of light generators (630, 632) oriented proximate to two opposing edges (640, 642) of the microscope slide (180, 502) so as to direct light to the two opposing edges (640,642) of the microscope slide (180, 502) whereby the light undergoes total internal reflection between the lower surface (242) of the microscope slide (180, 502) and a coverslip (182) covering the specimen situated on the microscope slide (180, 502), the light selected to have a wavelength designed to stimulate the specimen (220), wherein each of the pair of light generators (630,632) is an array of LED diodes (646, 648);
a camera (160);
an imaging lens (170) positioned to direct radiation emitted from the specimen (220) onto the camera (160);
wherein the total internal reflection of the light provides a spatially uniform distribution of light which causes a fluorescence emission (192) from the specimen (220), wherein the imaging system (100) comprises the microscope slide (180,502).

2. The imaging system of claim 1, wherein the LED diodes (646,648) are ultraviolet LED diodes.

3. The imaging system of any of claim 1 or 2, wherein two or more fluorophores are bound to the tissue and wherein the light source (222) includes one or more light sources configured to emit light at one or more wavelengths to stimulate each of the two or more fluorophores.

4. A microscope incorporating the imaging system (100) of any of claims 1 to 3.

5. A method for imaging a specimen (220) located on the upper surface (240) of a microscope slide (180, 614), the method comprising:
directing light into two opposing edges (640, 642) of the microscope slide (180, 614) at an angle sufficient to trigger total internal reflection of the light between the microscope slide (180, 614) and a coverslip (182, 503) covering the specimen (220) using a light source (222), wherein the light source (222) comprises a pair of light generators (630, 632) oriented proximate to the two opposing edges (640,642), wherein each of the light generators (630,632) is an array of LED diodes (646, 648), wherein the total internal reflection of the light provides a spatially uniform distribution of light which causes a fluorescence emission (192) from the specimen (220);
directing light (192) emitted from the specimen (220) towards a camera (160); and
generating an image of the microscope slide (180, 614) with the camera (160).

6. The method of claim 5, further comprising capturing in a single image the entire microscope slide (180, 614).

7. The method of claim 5 or 6, wherein directing the light into the two opposing edges of the microscope slide (180,614) includes directing ultraviolet light towards the two opposing edges (640,642).

8. The method of any of claims 5 to 7, wherein the specimen (222) is tissue, and the method further comprising stimulating at least one of a fluorophore, chromogen, or naturally-occurring molecule associated with the tissue located between the microscope slide (180,614) and coverslip (182,503) to emit the light from the specimen (220).

9. The method of any of claims 5 to 8, wherein the method further comprises blocking light from the light source (222) from directly impinging on the camera (160).

10. The method of any of claims 5 to 9, wherein the specimen (220) is tissue, and wherein directing the light into the two opposing edges (640,642) of the microscope slide (180,614) includes:
directing light at a first wavelength or a first waveband to stimulate a first fluorophore bound to the tissue situated on the slide (180,614); and
directing light at a second wavelength or a second waveband to stimulate a second fluorophore bound to the tissue.

11. The method of any of claims 5 to 10, wherein generating the image of the slide (180,614) with the camera (160) includes capturing in a single image of substantially the entire microscope slide (180,614).

## Patentansprüche

1. Abbildungssystem (100) für eine Gewebeerkennung an einer Probe (220), die auf einem Mikroskop-Objektträger (180, 502) liegt, der eine Oberseite (240), eine Unterseite (242) und mehrere Ränder aufweist, wobei die Probe (220) auf der Oberseite (240) liegt, wobei das Abbildungssystem (100) umfasst:
eine Lichtquelle (222), die zwei ein Paar bildende Lichtgeneratoren (630, 632) umfasst, die nahe an zwei einander entgegengesetzten Rändern (640, 642) des Mikroskop-Objektträgers (180, 502) ausgerichtet sind, so dass sie Licht auf die zwei einander entgegengesetzten Ränder (640, 642) des Mikroskop-Objektträgers (180, 502) richten, wobei das Licht eine Totalreflexion zwischen der Unterseite (242) des Mikroskop-Objektträgers (180, 502) und einem Deckglas (182), das die auf dem Mikroskop-Objektträger (180, 502) liegende Probe abdeckt, durchmacht, wobei das Licht so ausgewählt wird, dass es eine Wellenlänge aufweist, die dafür ausgelegt ist, die Probe (220) zu stimulieren, wobei jeder von den zwei Lichtgeneratoren (630, 632) ein Array von LED-Dioden (646, 648) ist;
eine Kamera (160);
eine Abbildungslinse (170), die so positioniert ist, dass sie eine Strahlung, die aus der Probe (220) emittiert wird, auf die Kamera (160) richtet;
wobei die Totalreflexion des Lichts für eine räumlich gleichmäßige Lichtverteilung sorgt, die eine Fluoreszenzemission (192) aus der Probe (220) bewirkt, wobei das Abbildungssystem (100) den Mikroskop-Objektträger (180, 502) umfasst.

2. Abbildungssystem nach Anspruch 1, wobei die LED-Dioden (646, 648) ultraviolette LED-Dioden sind.

3. Abbildungssystem nach einem von Anspruch 1 und 2, wobei zwei oder mehr Fluorophore an das Gewebe gebunden sind und wobei die Lichtquelle (222) eine oder mehrere Lichtquellen umfasst, die dafür ausgelegt ist bzw. sind, Licht mit einer oder mehreren Wellenlängen zu emittieren, um jeden der zwei oder mehr Fluorophore zu stimulieren.

4. Mikroskop, das Abbildungssystem (100) nach einem der Ansprüche 1 bis 3 enthaltend.

5. Verfahren zum Abbilden einer Probe (220), die auf der Oberseite (240) eines Mikroskop-Objektträgers (180, 614) liegt, wobei das Verfahren umfasst:
Richten von Licht auf zwei einander entgegengesetzte Ränder (640, 642) des Mikroskop-Objektträgers (180, 614) in einem Winkel, der ausreicht, um eine Totalreflexion des Lichts zwischen dem Mikroskop-Objektträger (180, 614) und einem Deckglas (182, 503), das die Probe (220) abdeckt, auszulösen, unter Verwendung einer Lichtquelle (222), wobei die Lichtquelle (222) ein Paar Lichtgeneratoren (630, 632) umfasst, die nahe an den zwei einander entgegengesetzten Rändern (640, 642) ausgerichtet sind, wobei jeder von den Lichtgeneratoren (630, 632) ein Array von LED-Dioden (646, 648) ist, wobei die Totalreflexion des Lichts für eine räumlich gleichmäßige Lichtverteilung sorgt, die eine Fluoreszenzemission (192) aus der Probe (220) bewirkt;
Richten von Licht (192), das aus der Probe (220) emittiert wird, auf eine Kamera (160); und
Erzeugen eines Bildes des Mikroskop-Objektträgers (180, 614) mit der Kamera (160).

6. Verfahren nach Anspruch 5, ferner das Aufnehmen des gesamten Mikroskop-Objektträgers (180, 614) in einem einzigen Bild umfassend.

7. Verfahren nach Anspruch 5 oder 6, wobei das Richten des Lichts auf die zwei einander entgegengesetzten Ränder des Mikroskop-Objektträgers (180, 614) das Richten von ultraviolettem Licht auf die zwei einander entgegengesetzten Ränder (640, 642) umfasst.

8. Verfahren nach einem der Ansprüche 5 bis 7, wobei die Probe (222) Gewebe ist und das Verfahren ferner das Stimulieren eines Fluorophors und/oder eines Chromogens und/oder eines in der Natur vorkommenden Moleküls, das mit dem zwischen dem Mikroskop-Objektträger (180, 614) und dem Deckglas (182, 503) liegenden Gewebe assoziiert ist, umfasst, um das Licht aus der Probe (220) zu emittieren.

9. Verfahren nach einem der Ansprüche 5 bis 8, wobei das Verfahren ferner einen direktes Einfall von Licht aus der Lichtquelle (222) auf die Kamera (160) blockiert.

10. Verfahren nach einem der Ansprüche 5 bis 9, wobei die Probe (220) Gewebe ist, und wobei das Richten des Lichts auf die zwei einander entgegengesetzten Ränder (640, 642) des Mikroskop-Objektträgers (180, 614) beinhaltet:
Richten von Licht mit einer ersten Wellenlänge oder einem ersten Wellenband zum Stimulieren eines ersten Fluorophors, der an das Gewebe gebunden ist, das sich auf dem Objektträger (180, 614) befindet; und
Richten von Licht mit einer zweiten Wellenlänge oder einem zweiten Wellenband zum Stimulieren eines zweiten Fluorophors, der an das Gewebe gebunden ist.

11. Verfahren nach einem der Ansprüche 5 bis 10, wobei das Erzeugen des Bildes des Objektträgers (180, 614) mit der Kamera (160) das Aufnehmen von im Wesentlichen dem gesamten Mikroskop-Objektträger (180, 614) in einem einzigen Bild umfasst.

## Revendications

1. Système d'imagerie (100) pour une détection de tissu d'un échantillon (220) situé sur une platine porte-objet de microscope (180, 502) ayant une surface supérieure (240), une surface inférieure (242), et une pluralité de bords, l'échantillon (220) est situé sur la surface supérieure (240), le système d'imagerie (100) comprenant :
une source de lumière (222) comprenant une paire de générateurs de lumière (630, 632) orientés à proximité de deux bords opposés (640, 642) de la platine porte-objet de microscope (180, 502) de sorte à diriger de la lumière vers les deux bords opposés (640, 642) de la platine porte-objet de microscope (180, 502), ce par quoi la lumière subit une réflexion totale interne entre la surface inférieure (242) de la platine porte-objet de microscope '180, 502) et un couvre-objet (182) recouvrant l'échantillon situé sur la platine porte-objet de microscope (180, 502), la lumière étant choisie pour avoir une longueur d'onde prévue pour stimuler l'échantillon (220), où chacune des paires de générateurs de lumière (630, 632) est un réseau de diodes DEL (646, 648) ;
une caméra (160) ;
une lentille d'imagerie (170) positionnée pour diriger un rayonnement émis à partir de l'échantillon (220) sur la caméra (160) ;
où la réflexion totale interne de la lumière procure une distribution spatialement uniforme de lumière qui provoque une émission de fluorescence (192) à partir de l'échantillon (220), où le système d'imagerie (100) comprend la platine porte-objet de microscope (180, 502).

2. Système d'imagerie selon la revendication 1, dans lequel les diodes DEL (646, 648) sont des diodes DEL ultraviolettes.

3. Système d'imagerie selon l'une quelconque des revendications 1 ou 2, dans lequel deux ou davantage de photophores sont reliés au tissu et dans lequel la source de lumière (222) comporte une ou plusieurs sources de lumière configurées pour émettre de la lumière à une ou à plusieurs longueurs d'onde pour stimuler chacun parmi les deux ou davantage de photophores.

4. Microscope incorporant le système d'imagerie (100) selon l'une quelconque des revendications 1 à 3.

5. Procédé d'imagerie d'un échantillon (220) situé sur la surface supérieure (240) d'une platine porte-objet de microscope (180, 614), le procédé comprenant :
l'orientation de la lumière dans deux bords opposés (640, 642) de la platine porte-objet de microscope (180, 614) à un angle suffisant pour déclencher une réflexion totale interne de la lumière entre la platine porte-objet de microscope (180, 614) et un couvre-objet (182, 503) recouvrant l'échantillon (220) en utilisant une source de lumière (222), où la source de lumière (222) comprend une paire de générateurs de lumière (630, 632) orientés à proximité des deux bords opposés (640, 642), où chacun des générateurs de lumière (630, 632) est un réseau de diodes DEL (646, 648), où la réflexion totale interne de la lumière procure une distribution spatialement uniforme de lumière qui provoque une émission de fluorescence (192) à partir de l'échantillon (220) ;
l'orientation de la lumière (192) émise à partir de l'échantillon (220) vers une caméra (160) ; et
la génération d'une image de la platine porte-objet de microscope (180, 614) avec la caméra (160).

6. Procédé selon la revendication 5, comprenant en outre la capture en une seule image de la platine porte-objet de microscope (180, 614) entière.

7. Procédé selon la revendication 5 ou la revendication 6, dans lequel l'orientation de la lumière dans les deux bords opposés de la platine porte-objet de microscope (180, 614) comporte l'orientation de la lumière ultraviolette vers les bords opposés (640, 642).

8. Procédé selon l'une quelconque des revendications 5 à 7, dans lequel l'échantillon (222) est du tissu, et le procédé comprenant en outre une stimulation d'au moins élément parmi un fluorophore, un chromogène ou une molécule d'origine naturelle associée avec le tissu situé entre la platine porte-objet de microscope (180, 614) et le couvre-objet (182, 503) pour émettre la lumière à partir de l'échantillon (220).

9. Procédé selon l'une quelconque des revendications 5 à 8, où le procédé comprend en outre le blocage de la lumière à partir de la source de lumière (222) pour qu'elle n'empiète pas directement sur la caméra (160).

10. Procédé selon l'une quelconque des revendications 5 à 9, dans lequel l'échantillon (220) est du tissu, et dans lequel l'orientation de la lumière dans les deux bords opposés (640, 642) de la platine porte-objet de microscope (180, 614) comporte :
l'orientation de la lumière à une première longueur d'onde ou à une première bande d'ondes pour stimuler un premier fluorophore lié au tissu situé sur la platine porte-objet (180, 614) ; et
l'orientation de la lumière à une seconde longueur d'onde ou à une seconde bande d'ondes pour stimuler un second fluorophore lié au tissu.

11. Procédé selon l'une quelconque des revendications 5 à 10, dans lequel la génération de l'image de la platine porte-objet (180, 614) avec la caméra (160) comprend la capture en une seule image de la platine porte-objet de microscope (180, 614) pratiquement entière.
